# EUROPEAN PATENT APPLICATION

(11) **EP 4 372 750 A1**
(43) Date of publication of application: **22.05.2024**
(21) Application number: 23204631.8
(22) Date of filing: 19.10.2023
(51) Int. Cl.: G16B 15/30, G16B 25/10, G16B 40/20, G16C 20/50

(54) **METHOD AND SYSTEM FOR GENE EXPRESSION PREDICTION-BASED SCREENING OF DRUG-LIKE MOLECULES**

(30) Priority: 21.11.2022 IN 202221066851
(71) Applicant: Tata Consultancy Services Limited, Maharashtra (IN)
(72) Inventor: CHAKRABARTY, Broto, 500081 Hyderabad, Telangana (IN); PADHI, Siladitya, 500081 Hyderabad, Telangana (IN); SADRANI, Riya Dilipbhai, 560066 Bangalore, Karnataka (IN); SRINIVASAN, Rajgopal, 500081 Hyderabad, Telangana (IN); ROY, Arijit, 500081 Hyderabad,Telangana (IN)
(74) Representative: Goddar, Heinz J.

(57) **Abstract**

Traditional drug discovery methods are target-based, time- and resource-intensive, and require a lot of resources for the initial hit molecule identification. Phenotype-based drug screening requires differential gene expression data of a large number of molecules for different combinations of cell-line, time point and dosage. Experimentally obtaining gene expression data for all these combinations is again a heavily resource-intensive process. The technical challenge in conventional methods that use prediction models is that they depend largely on the data processing and representation. The disclosure herein generally relates to drug-like molecule screening, and, more particularly, to a method and system for gene expression and machine learning-based drug screening. The embodiment, thus, provides a mechanism of a small molecule-induced gene expression prediction based on machine learning models. Moreover, the embodiments herein further provide a mechanism of screening of drug-like molecules using the machine learning model(s).

## Description

### CROSS-REFERENCE TO RELATED APPLICATIONS AND PRIORITY

The present application claims priority to Indian application no. 202221066851, filed on November 21, 2022.

### TECHNICAL FIELD

The disclosure herein generally relates to screening of drug-like molecules, and, more particularly, to a method and system for machine learning based screening of potential drugs.

### BACKGROUND

Traditional drug discovery methods are target-based. They are time and resource intensive processes because a lot of resources are required for the initial hit molecule identification. Phenotype-based drug screening requires differential gene expression data of a large number of molecules for different combinations of cell-line, time point and dosage. Experimentally obtaining gene expression data for all these combinations is again a heavily resource intensive process requiring large amount of time and cost. Computational prediction models overcome these factors and provide an alternative for initial screening of drug-like small molecules. The technical challenge in conventional methods that use prediction models is that they depend largely on the data processing and representation.

### SUMMARY

Embodiments of the present disclosure present technological improvements as solutions to one or more of the above-mentioned technical problems recognized by the inventors in conventional systems. For example, in one embodiment, a processor implemented method is provided. In this method, initially a plurality of small molecules and a reference dataset comprising gene expression profiles are received, via one or more hardware processors, as input data. Further, a plurality of molecular representations are generated, via the one or more hardware processors, for the plurality of small molecules. Further, a Characteristic Distribution (CD) profile of a plurality of gene expressions in the reference dataset is generated. Further, a p-value of the CD profiles of the plurality of gene expressions is calculated, via the one or more hardware processors, wherein the p-value represents quality of the CD profiles. Further, a plurality of CD profiles having the p-value exceeding a threshold of p-value are filtered to obtain a plurality of high quality CD profiles of gene expressions, via the one or more hardware processors. Further, one or more machine learning models are generated using the plurality of molecular representations and the filtered CD profiles as training data, via the one or more hardware processors.

In another aspect, one of the machine learning models that has been identified as a best performing model is used for screening a plurality of drug-like molecules. The screening includes the following steps. Initially, a dataset comprising a plurality of molecules is received. Further, the CD profile representing gene expression of each of the plurality of molecules is predicted using the best performing model. Further, a reversibility score between each of the predicted CD profiles and a plurality of disease signature expression profiles is determined. Further, one or more molecules are selected from among the plurality of molecules as potential drug-like molecules to treat one or more diseases having a signature among the plurality of disease signature expression profiles, if the determined reversibility score between associated CD profile and disease signature is exceeding a threshold of reversibility score.

In yet another aspect, a system is provided. The system includes one or more hardware processors, a communication interface, and a memory storing a plurality of instructions. The plurality of instructions cause the one or more hardware processors to receive a plurality of small molecules and a reference dataset comprising gene expression profiles, as input data. Further, a plurality of molecular representations are generated, via the one or more hardware processors, for the plurality of small molecules. Further, a Characteristic Distribution (CD) profile of a plurality of gene expressions in the reference dataset is generated. Further, a p-value of the CD profiles of the plurality of gene expressions is calculated, via the one or more hardware processors, wherein the p-value represents quality of the CD profiles. Further, a plurality of CD profiles having the p-value lower than a threshold of p-value are filtered to obtain a plurality of high quality CD profiles of gene expressions, via the one or more hardware processors. Further, one or more machine learning models are generated using the plurality of molecular representations and the filtered CD profiles as training data, via the one or more hardware processors.

In yet another aspect, the system identifies one of the machine learning models that has been identified as a best performing model for screening a plurality of drug-like molecules, by executing the following steps. Initially, a dataset comprising a plurality of drug-like molecules is received. Further, the CD profile representing gene expression of each of the plurality of drug-like molecules is predicted using the best performing model. Further, a reversibility score between each of the predicted CD profiles and a plurality of disease signature expression profiles is determined. Further, one or more molecules are selected from among the plurality of drug-like molecules as potential drugs to treat one or more diseases having a signature among the plurality of disease signature expression profiles, if the determined reversibility score between associated CD profile and disease signature is exceeding a threshold of reversibility score.

In yet another aspect, a non-transitory computer readable medium is provided. The non-transitory computer readable medium includes a plurality of instructions, which when executed, cause the one or more hardware processors to initially receive a plurality of small molecules and a reference dataset comprising gene expression profiles as input data. Further, a plurality of molecular representations are generated, via the one or more hardware processors, for the plurality of small molecules. Further, a Characteristic Distribution (CD) profile of a plurality of gene expressions in the reference dataset is generated. Further, a p-value of the CD profiles of the plurality of gene expressions is calculated, via the one or more hardware processors, wherein the p-value represents quality of the CD profiles. Further, a plurality of CD profiles having the p-value exceeding a threshold of p-value are filtered to obtain a plurality of high quality CD profiles of gene expressions, via the one or more hardware processors. Further, one or more machine learning models are generated using the plurality of molecular representations and the filtered CD profiles as training data, via the one or more hardware processors.

In yet another aspect, the non-transitory computer readable medium causes the one or more hardware processors to identify one of the machine learning models that has been identified as a best performing model and further use it for screening a plurality of drug-like molecules. The screening includes the following steps. Initially, a dataset comprising a plurality of small molecules is received. Further, the CD profile representing gene expression of each of the plurality of small molecules is predicted using the best performing model. Further, a reversibility score between each of the predicted CD profiles and a plurality of disease signature expression profiles is determined. Further, one or more molecules are selected from among the plurality of small molecules as potential drug-like molecules to treat one or more diseases having a signature among the plurality of disease signature expression profiles, if the determined reversibility score between associated CD profile and disease signature is exceeding a threshold of reversibility score.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary system for generating a data model ad for screening drugs, according to some embodiments of the present disclosure.
FIG. 2 is a flow diagram depicting steps involved in the process of generating a machine learning model for screening drugs, by the system of FIG. 1, according to some embodiments of the present disclosure.
FIG. 3 is a flow diagram depicting steps involved in the process of screening drugs using the machine learning model, by the system of FIG. 1, according to some embodiments of the present disclosure.
FIGS. 4, and 5, depict example figures supporting experimental data, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Traditional drug discovery methods are target-based. They are time and resource intensive processes because a lot of resources are required for the initial hit molecule identification. Phenotype-based drug screening requires differential gene expression data of a large number of molecules for different combinations of cell-line, time point and dosage. Experimentally obtaining gene expression data for all these combinations is again a heavily resource intensive process requiring large amount of time and cost. Computational prediction models overcome these factors and provide an alternative for initial screening of drug-like small molecules. The technical challenge in conventional methods that use prediction models is that they depend largely on the data processing and representation.

Method and system disclosed herein provide a small molecule-induced gene expression prediction based approach for generating machine learning models. From among the machine learning models built, one machine learning model is identified as best performing model, which is further used to receive and process a dataset comprising a plurality of drug-like molecules, and perform screening of drugs.

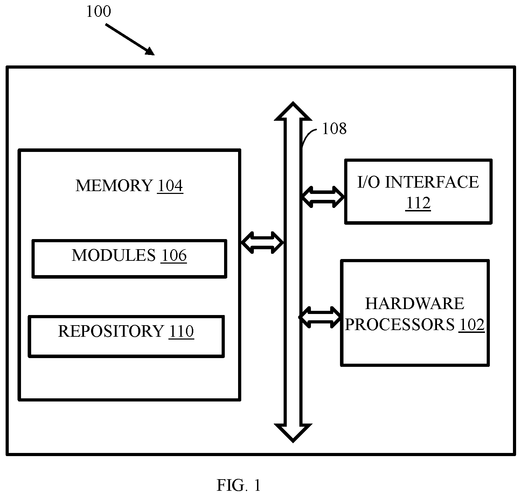

Referring now to the drawings, and more particularly to FIG. 1 through FIG. 5, where similar reference characters denote corresponding features consistently throughout the figures, there are shown preferred embodiments and these embodiments are described in the context of the following exemplary system and/or method.

FIG. 1 illustrates an exemplary system for generating a data model and for screening drugs, according to some embodiments of the present disclosure. The system 100 includes or is otherwise in communication with hardware processors 102, at least one memory such as a memory 104, an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable the system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in the memory 104.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 104 includes a plurality of modules 106.

The plurality of modules 106 include programs or coded instructions that supplement applications or functions performed by the system 100 for executing different steps involved in the process of switching between hardware accelerators for model training, being performed by the system 100. The plurality of modules 106, amongst other things, can include routines, programs, objects, components, and data structures, which performs particular tasks or implement particular abstract data types. The plurality of modules 106 may also be used as, signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for the switching between hardware accelerators for model training.

The data repository (or repository) 110 may include a plurality of abstracted piece of code for refinement and data that is processed, received, or generated as a result of the execution of the plurality of modules in the module(s) 106.

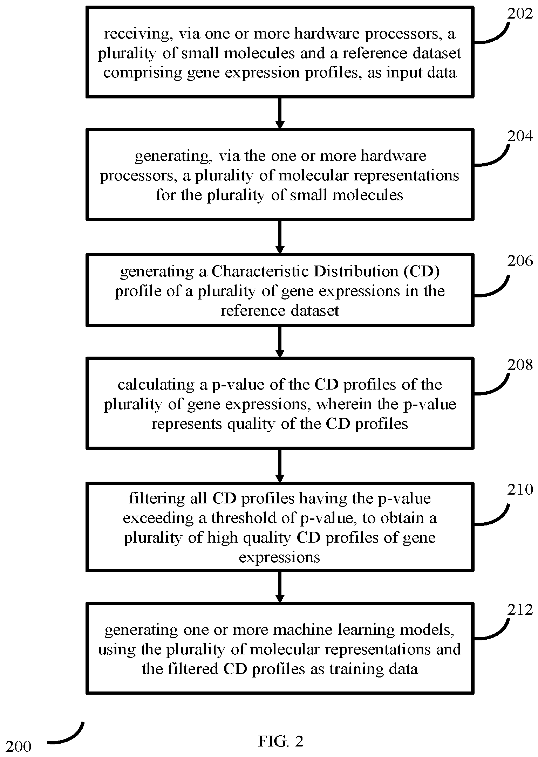

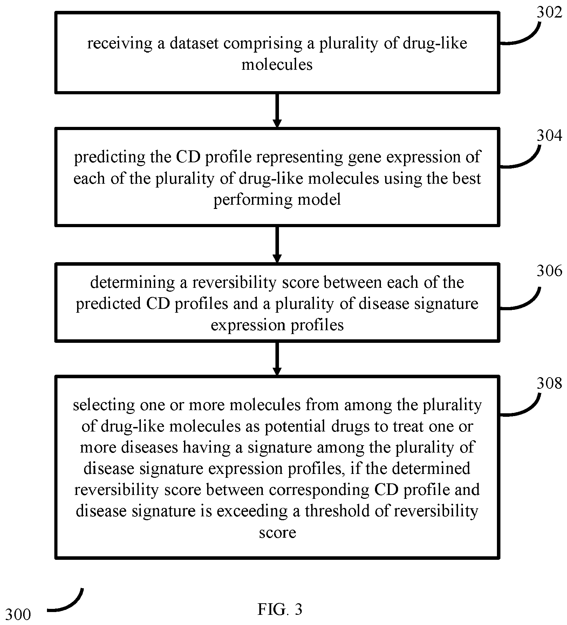

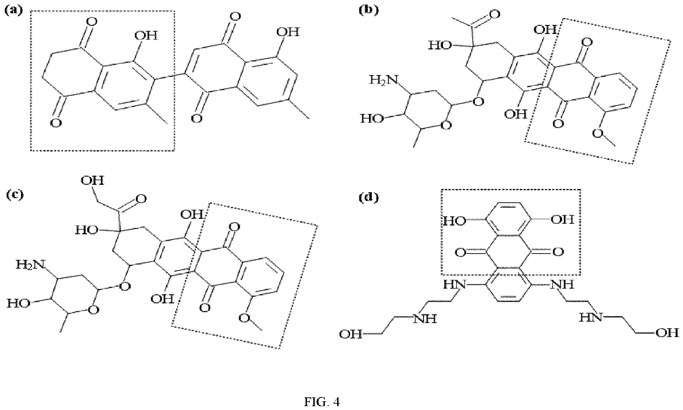

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the system 100 are now explained with reference to the steps in flow diagrams in FIG. 2 and FIG. 3, and the example diagrams in FIGS. 4 through 6.

FIG. 2 is a flow diagram depicting steps involved in the process of generating a machine learning model for screening drugs, by the system of FIG. 1, according to some embodiments of the present disclosure. In an embodiment, the system 100 comprises one or more data storage devices or the memory 104 operatively coupled to the processor(s) 102 and is configured to store instructions for execution of steps of the method 200 by the processor(s) or one or more hardware processors 102. The steps of the method 200 of the present disclosure will now be explained with reference to the components or blocks of the system 100 as depicted in FIG. 1 and the steps of flow diagram as depicted in FIG. 2. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

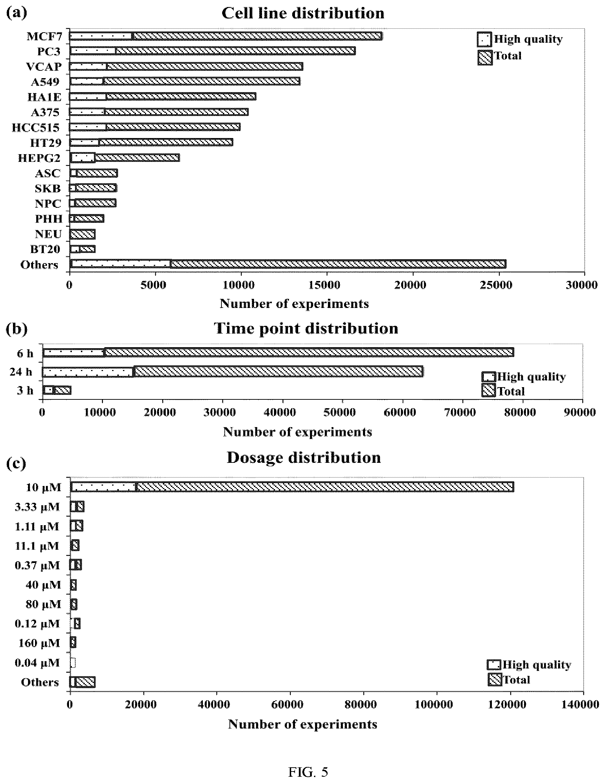

At step 202 of method 200 in FIG. 2, the system 100 receives, from one or more associated sources, a plurality of small molecules and a reference dataset comprising gene expression profiles, via the one or more hardware processors 102, as input data. Small molecule is a term used to indicate organic compound having low molecular weight (≤ 1000 daltons) that may regulate a biological process, with a size on the order of 1 nm. The reference database used maybe library of integrated network-based cellular signatures (LINCS) L1000 that provides differential gene expression profiles of chemically perturbed human cell lines based on Luminex bead technology. Further, at step 204 of the method 200, the system 100 generates a plurality of molecular representations, via the one or more hardware processors, for the plurality of small molecules. Molecular representation reduces the dimensionality of a molecular structure into a chemically meaningful format that relays important chemical information. The system 100 generates the molecular representations by representing the plurality of small molecules using one of a Simplified Molecular-Input Line-Entry System (SMILES) and an Extended-Connectivity Fingerprints (ECFPs). In an embodiment, any other suitable technique maybe used. Similarly, the gene expression profiles are generated by administering the plurality of small molecules on different cell lines with different concentrations, measured at different time instances. Distribution of small molecule induced gene expression data across a) cell line, b) time points, and c) small molecule dose, are depicted in FIG. 5 (a), (b), and (c) respectively.

Further, at step 206 of the method 200, the system 100 generates a Characteristic Distribution (CD) profile of a plurality of gene expressions in the reference dataset. The CD uses geometrical separation between the two groups to identify significant genes. An example of a subset of the CD profile, the molecule Cosmosiin (BRD-K13642330) on VCAP cell line for 10µM concentration dose and 6hrs of exposure is given in Table. 1 below:

**Table. 1**

| **Gene ID** | **Gene Name** | **Characteristic Direction** |
|---|---|---|
| 5720 | PSME1 | -0.0142 |
| 466 | ATF1 | -0.0167 |
| 6009 | RHEB | 0.0037 |
| 2309 | FOXO3 | -0.0187 |
| 387 | RHOA | -0.0166 |
| 3553 | IL1B | 0.0082 |
| 427 | ASAH1 | -0.0172 |
| 5898 | RALA | 0.0206 |
| 23365 | ARHGEF12 | -0.0376 |
| 6657 | SOX2 | 0.0276 |
| 5054 | SERPINE1 | -0.0407 |
| 3108 | HLA-DMA | -0.0178 |
| 1950 | EGF | 0.0114 |
| 351 | APP | -0.0047 |
| 4846 | NOS3 | 0.0363 |
| 1452 | CSNK1A1 | -0.0011 |
| 4776 | NFATC4 | -0.0087 |
| 6908 | TBP | -0.0084 |
| 672 | BRCA1 | -0.0305 |
| 5710 | PSMD4 | -0.0161 |
| 2115 | ETV1 | -0.0108 |
| 7015 | TERT | 0.0054 |
| 8726 | EED | -0.0170 |

Further, at step 208 of the method 200, the system 100 calculates a p-value of the CD profiles of the plurality of gene expressions, via the one or more hardware processors 102, wherein the p-value represents quality of the CD profiles. In an embodiment, the system 100 may use any suitable technique such as but not limited to Fisher's exact test, to calculate the p-value. Lower p-values indicate that consistent differential expressions were observed across all replicates for which expression values were considered for calculating the p-values. Further, at step 210 of the method 200, the system 100 filters a plurality of CD profiles having the p-value lower than a threshold of p-value, and the CD profiles that remain, which have p-value lower than the threshold of p-value, are considered to be high quality profiles of gene expression, and are obtained by the system 100 in this manner, via the one or more hardware processors 102. For example, the p-value used is 0.05. Further, at step 212 of the method 200, the system 100 generates one or more machine learning models using the plurality of molecular representations and the filtered CD profiles as training data, via the one or more hardware processors 102. The machine learning models are generated to predict the gene expression profile corresponding to a small molecule perturbagen, cell type, dosage and time of exposure. Different types of machine learning models, such as but not limited to Decision Tree, Random Forest, K-Nearest Neighbors, Linear Regression, Ridge Regressor, Lasso Regressor, Vanilla Neural Network, and Graph Convolutional Network maybe generated.

From among the plurality of machine learning models, a model having highest performance is identified as a best performing model. In an embodiment, the performance of each of the plurality of models is determined with respect to capability to correctly predict gene expression profile for known drug-like molecules, for a test data or for a portion of real-time data input. In another aspect, one of the machine learning models that has been identified as a best performing model is used for screening a plurality of drug-like molecules. The screening includes the following steps, which are depicted in method 300 in FIG. 3. At step 302 of the method 300, a dataset comprising a plurality of molecules is received. Further, at step 304 of the method 300, the system 100 predicts, using the best performing model, the CD profile representing gene expression of each of the plurality of molecules. At this stage, for generating predictions, the system 100 uses small molecule information in a suitable format (for example, SMILES format), cell type, dosage and time point, as inputs. Further, a reversibility score between each of the predicted CD profiles and a plurality of disease signature expression profiles is determined. Consider the example in FIG. 4. In this example, Beta'-dihydrodiospyrin belongs to the class of naphthoquinones, sibiriquinone B belongs to diterpenoids and chaetoindicin B belongs to aryl ketones. All of these classes were shown to exhibit anti-cancer activity in studies conducted. Beta'-dihydrodiospyrin contains the naphthoquinone moiety, which is present in known anti-cancer drugs like daunorubicin, doxorubicin and mitoxantrone. FIG. 4 shows the naphthoquinone moiety in beta'-dihydrodiospyrin and known anti-cancer drugs daunorubicin, doxorubicin and mitoxantrone. Interestingly, beta'-dihydrodiospyrin is a dimeric naphthoquinone, which is a class of compounds known to exhibit prostate cancer cytotoxicity. In summary, the approach described here has made it possible to screen a database of NPs and identify a select few NPs that may show anti-cancer activity. The approach disclosed herein thus can be used to screen libraries of molecules and identify molecules that are of therapeutic interest.

Against a given disease, in an embodiment, the reversibility score is determined as cosine distance between each of the predicted CD profiles and a plurality of disease signature expression profiles. The predicted small molecule-induced gene expression profile is compared with the differential gene expression profile of the disease state based on the reversibility score. The reversibility score captures the ability of the molecule to reverse the phenotypic effect of a disease. If the gene expression profile induced by a small molecule is the reverse of the gene expression profile corresponding to the disease, then the molecule's effect can take a diseased cell back to its healthy state. The reversibility score ranges between 0 to 2, where higher reversibility score indicates better drug. Thus, the reversibility score of a molecule for a particular disease state may be considered as a computational screening step to evaluate its phenotypic effectiveness.

Further, one or more molecules are selected from among the plurality of drug-like molecules as potential drugs to treat one or more diseases having a signature among the plurality of disease signature expression profiles, if the determined reversibility score between associated CD profile and disease signature is exceeding a threshold of reversibility score.

As this approach facilitates drug screening based on the phenotypic effect of the drug, it helps in analyzing the cellular dynamics of the effect of the drug as it considers several factors that determine the effect of a drug in a disease state.

### Experimental Data:

### A) Generating disease signatures

The cell lines considered in this study were MCF7, VCAP and PC3. VCAP and PC3 are prostate cancer cell lines while MCF7 is a breast cancer cell line. Therefore, the disease signatures were obtained for prostate and breast cancers. The Gene Expression Omnibus (GEO) repository was explored to identify suitable studies where high-throughput Ribonucleic acid (RNA) sequencing was performed to measure the differential gene expression of cancer cells. The experimental study with GEO id GSE133626 provides gene expression data for complete transcriptome of prostate cancer samples and matched control samples. This data was used to obtain the prostate cancer signature of differential gene expression using the characteristic direction method. Similarly, breast cancer signature was obtained from the RNA sequencing study with GEO id GSE60182.

### B) Screening conducted:

The LINCS L1000 repository stores the experimentally obtained gene expression profiles for small molecules that were administered on different cell lines with different concentrations and measured after different time points. The prediction models took the small molecule representation and time point as input, and predicted the small molecule-induced gene expression profile in the form of characteristic direction (CD). The small molecule-induced gene expression profiles in the form of CD were obtained from the L1000CDS2 portal. The models were trained separately for three different cell lines - MCF7, VCAP and PC3, keeping the dosage constant at 10 µM concentration.

The prediction models were developed for specific cell types and dosage. The models take the small molecule information in SMILES format, cell type, dosage and time point, as input. The output of the model is gene expression profile in the form of characteristic direction for 978 landmark genes. The predicted small molecule-induced gene expression profile is compared with the differential gene expression profile of the disease state based on the cosine distance, which is denoted as the `Reversibility score'. The reversibility score captures the ability of the drug to reverse the phenotypic effect of a disease. If the gene expression profile induced by a drug is the reverse of the gene expression profile corresponding to the disease, then the drug's effect can take a diseased cell back to its healthy state. The reversibility score ranges between 0 to 2, where higher reversibility score indicates better drug. Thus, the reversibility score of a molecule for a particular disease state may be considered as a computational screening step to evaluate its phenotypic effectiveness.

### C) Use case example of screening natural products to identify potential anti-cancer drugs

The use case for screening natural products is based on screening of the COCONUT natural product database to find anti-prostate cancer molecules. All natural products that had an annotation level of 5 (i.e., fully annotated) were extracted for screening; this resulted in ~60K natural products. Characteristic direction profiles corresponding to the extracted natural products were predicted for the VCAP cell line using the random forest model. VCAP, being a prostate cancer cell line, was chosen here since the screening was to be performed for identifying anti-prostate cancer molecules. Upon obtaining the predicted characteristic direction profiles for the natural products, the ability of a natural product to reverse the state of a prostate cancer cell was quantified by means of a reversibility score. The reversibility score used in this example was the cosine distance between the predicted characteristic direction profile for the natural product and the disease signature profile for prostate cancer. The higher the reversibility score, the better the natural product is at reversing the disease state. Thus, the natural products were sorted based on their reversibility score. Among the top ten natural products obtained after the sorting, three belonged to chemical classes that have previously been shown to exhibit anti-cancer activity. Furthermore, one of the natural products contained a moiety that is known to occur in certain anti-prostate cancer drugs. Thus, the method 200 was capable of identifying potent anti-prostate cancer natural products from a library of ~60K molecules.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problem of screening of drugs. The embodiment, thus provides a mechanism of a small molecule-induced gene expression prediction based on machine learning models. Moreover, the embodiments herein further provide a mechanism of screening of drug-like molecules using the machine learning model(s). Graphical representation supporting data used in the experimental setup are depicted in FIGS. 4 through 6.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (200), comprising:
receiving (202), via one or more hardware processors, a plurality of small molecules and a reference dataset comprising gene expression profiles, as input data;
generating (204), via the one or more hardware processors, a plurality of molecular representations for the plurality of small molecules;
generating (206), via the one or more hardware processors, a Characteristic Distribution (CD) profile of a plurality of gene expressions in the reference dataset;
calculating (208), via the one or more hardware processors, a p-value of the CD profiles of the plurality of gene expressions, wherein the p-value represents quality of the CD profiles;
filtering (210), via the one or more hardware processors, a plurality of CD profiles having the p-value exceeding a threshold of p-value, to obtain a plurality of high quality CD profiles of gene expressions; and
generating (212), via the one or more hardware processors, one or more machine learning models, using the plurality of molecular representations and the filtered CD profiles as training data.

2. The processor implemented method as claimed in claim 1, wherein from among the one or more machine learning models a machine learning model having highest value of performance from among the one or more machine learning models is determined as a best performing model.

3. The processor implemented method as claimed in claim 2, wherein the best performing model is used for screening a plurality of drug-like molecules, comprising:
receiving (302) a dataset comprising a plurality of molecules;
predicting (304) the CD profile representing gene expression of each of the plurality of molecules using the best performing model;
determining (306) a reversibility score between each of the predicted CD profiles and a plurality of disease signature expression profiles; and
selecting (308) one or more molecules from among the plurality of molecules as potential drug like molecules to treat one or more diseases having a signature among the plurality of disease signature expression profiles, if the determined reversibility score between associated CD profile and disease signature is exceeding a threshold of reversibility score.

4. The processor implemented method as claimed in claim 1, wherein the gene expression profiles are obtained by administering the plurality of small molecules on different cell lines with different concentrations, measured at different time instances.

5. The processor implemented method as claimed in claim 1, wherein the molecular representations are obtained by representing the plurality of small molecules using one of a Simplified Molecular-Input Line-Entry System (SMILES) and an Extended-Connectivity Fingerprints (ECFPs).

6. A system (100), comprising:
one or more hardware processors (102);
a communication interface (112); and
a memory (104) storing a plurality of instructions, wherein the plurality of instructions cause the one or more hardware processors to:
receive a plurality of small molecules and a reference dataset comprising gene expression profiles, as input data;
generate a plurality of molecular representations for the plurality of small molecules;
generate a Characteristic Distribution (CD) profile of a plurality of gene expressions in the reference dataset;
calculate a p-value of the CD profiles of the plurality of gene expressions, wherein the p-value represents quality of the CD profiles;
filter a plurality of CD profiles having the p-value exceeding a threshold of p-value, to obtain a plurality of high quality CD profiles of gene expressions; and
generate one or more machine learning models, using the plurality of molecular representations and the filtered CD profiles as training data.

7. The system as claimed in claim 6, wherein the one or more hardware processors are configured to determine a machine learning model having a highest value of performance from among the one or more machine learning models as a best performing model.

8. The system as claimed in claim 7, wherein the one or more hardware processors are configured to screen a plurality of drug-like molecules using the best performing model, by:
receiving a dataset comprising a plurality of molecules;
predicting the CD profile representing gene expression of each of the plurality of molecules using the best performing model;
determining reversibility score between each of the predicted CD profiles and a plurality of disease signature expression profiles; and
selecting one or more molecules from among the plurality of molecules as potential drug-like molecules to treat one or more diseases having a signature among the plurality of disease signature expression profiles, if the determined reversibility score between associated CD profile and disease signature is exceeding a threshold of reversibility score.

9. The system as claimed in claim 6, wherein the one or more hardware processors are configured to obtain the gene expression profiles by administering the plurality of small molecules on different cell lines with different concentrations, measured at different time instances.

10. The system as claimed in claim 6, wherein the one or more hardware processors are configured to obtain the molecular representations by representing the plurality of small molecules using one of a Simplified Molecular-Input Line-Entry System (SMILES) and an Extended-Connectivity Fingerprints (ECFPs).

11. One or more non-transitory machine-readable information storage mediums comprising one or more instructions which when executed by one or more hardware processors cause:
receiving a plurality of small molecules and a reference dataset comprising gene expression profiles, as input data;
generating a plurality of molecular representations for the plurality of small molecules;
generating a Characteristic Distribution (CD) profile of a plurality of gene expressions in the reference dataset;
calculating a p-value of the CD profiles of the plurality of gene expressions, wherein the p-value represents quality of the CD profiles;
filtering a plurality of CD profiles having the p-value exceeding a threshold of p-value, to obtain a plurality of high quality CD profiles of gene expressions; and
generating one or more machine learning models, using the plurality of molecular representations and the filtered CD profiles as training data.

12. The one or more non-transitory machine-readable information storage mediums as claimed in claim 11, wherein from among the one or more machine learning models a machine learning model having highest value of performance from among the one or more machine learning models is determined as a best performing model.

13. The one or more non-transitory machine-readable information storage mediums as claimed in claim 12, wherein the best performing model is used for screening a plurality of drug-like molecules, comprising:
receiving a dataset comprising a plurality of molecules;
predicting the CD profile representing gene expression of each of the plurality of molecules using the best performing model;
determining a reversibility score between each of the predicted CD profiles and a plurality of disease signature expression profiles; and
selecting one or more molecules from among the plurality of molecules as potential drug like molecules to treat one or more diseases having a signature among the plurality of disease signature expression profiles, if the determined reversibility score between associated CD profile and disease signature is exceeding a threshold of reversibility score.

14. The one or more non-transitory machine-readable information storage mediums as claimed in claim 11, wherein the gene expression profiles are obtained by administering the plurality of small molecules on different cell lines with different concentrations, measured at different time instances.

15. The one or more non-transitory machine-readable information storage mediums as claimed in claim 11, wherein the molecular representations are obtained by representing the plurality of small molecules using one of a Simplified Molecular-Input Line-Entry System (SMILES) and an Extended-Connectivity Fingerprints (ECFPs).
